# EUROPEAN PATENT APPLICATION

(11) **EP 2 431 010 A1**
(43) Date of publication of application: **21.03.2012**
(21) Application number: 10176941.2
(22) Date of filing: 15.09.2010
(51) Int. Cl.: A61F 7/12

(54) **Apparatus for performing heat treatment**

(71) Applicant: Wallsten, Hans I., 1135 Denens (CH)
(72) Inventor: Wallsten, Hans I., 1135 Denens (CH)
(74) Representative: Somlo, Tommy

(57) **Abstract**

The present invention relates to an apparatus for performing heat treatment of a bodily cavity in a mammal. The apparatus comprises a catheter intended for insertion into said bodily cavity, an inflatable balloon surrounding a distal end of said catheter in a liquid tight manner which balloon is adapted in use of said apparatus to be inflated and dilated in said bodily cavity, control means operatively connected to said balloon by at least one oscillation lumen and being provided with an oscillation assembly adapted to drive a heat transmitting fluid within said apparatus, and heating means adapted to heat said heat transmitting fluid. Said control means are adapted to control pressure and temperature of said heat transmitting fluid. The heating means have a heat emitting portion and a heat receiving portion which is use of the apparatus are positioned in thermal contact with each other, and which are separable from each other.

## Description

### Field of the invention

The present invention relates to an apparatus for performing heat treatment of a bodily cavity in a mammal. The apparatus comprises a catheter intended for insertion into said bodily cavity, an inflatable balloon surrounding a distal end of said catheter in a liquid tight manner which balloon is adapted in use of said apparatus to be inflated and dilated in said bodily cavity, control means operatively connected to said balloon by at least one oscillation lumen and being provided with an oscillation assembly adapted to drive a heat transmitting fluid within said apparatus, and heating means adapted to heat said heat transmitting fluid, said control means being adapted to control pressure and temperature of said heat transmitting fluid.

### Technical background

Heavy menstrual bleeding (menorrhagia) is a very common problem for women, often occurring when a woman reaches the mid-thirties or early forties. Menorrhagia has been treated by hysterectomy which is a surgical method to remove the uterus. It is major surgery involving serious risks and high costs for medical care. A new simpler method was developed at the last decade of the 20th century using hot balloon dilation of the uterus cavity causing necrosis and ablation of the inner lining of the endometrium ending the bleeding.

The method is simple and fast and do not require a highly skilled surgeon. Today is therefore hot balloon dilation used in an increasing number of treatments and several 100.000 procedures annually performed in the western world.

Hot balloon dilation of uterus is performed by the use of a balloon catheter attached to a control unit. A deflated balloon in the distal end of the catheter is introduced through the cervix into the uterine cavity, where it is inflated and pressurised by a hot liquid during several minutes for destruction of the inner layer of the endometrium. The control unit is providing energy for the heating and pressure. The pressurised balloon reduces the cooling effect of the blood perfusion improving the necrotising.

Hot balloon catheters for the treatment of menorrhagia are described for example in US patent 4,949,718 (Neuwirth et al), US patent RE 37,651 (Wallstén), WO96/26695 (Clarén). In US 4,949,718 (Neuwirth) is a heater in form of electric resistance elements located in the middle of the balloon. The catheter, which is sterile, is attached to the non-sterile control unit providing i.a. electric power for the heater. The sterile catheter is generally disposed after use.

The RE 37,651 (Wallstén)-document contains an important feature in form of a forced circulation through a heater in a balloon. The circulation which considerably increases the heat transfer to the endometrium gives a more even necrotisation and also shortening the treatment time. The circulation is achieved by first means located in the sterile catheter, which is attached to the non-disposable part in which second mechanical means are located causing a back and forth movement of the first means and consequently of the liquid in the catheter. Two counter acting back valves in the balloon will transform the back and forth movement to a violent circulation of the liquid through the heater in the balloon. The non-disposable part also provides electric power for the heater, which may be a PTC-element.

In WO96/26695 (Clarén) is a balloon catheter used, which is attached to a tank filled with liquid and to a system consisting of a peristatic pump for the circulation of the liquid between the tank and the balloon. This device has several disadvantages compared to the Wallstén device earlier described. There is a long heating up time for the large amount of liquid and a risk for contamination of the pump system and the large amount of liquid in the tank as it is used several times. Furthermore a burst balloon would cause large amounts of pressurised very hot liquid to flow out from the uterus causing damages on the patient.

The Wallstén device has been improved during the years and is marketed under the name of Cavaterm plus™. The improved device is described in the patent EP 1 139 944 B1 (Wallstèn et al), even if the claims are directed towards means to adapt the balloon length to different cavity sizes. Of particular interest are the figures 4 and 5 with the corresponding description, which are incorporated by reference. In the shown embodiment is a heater and two counter acting valves incorporated in the handle of the catheter.

There is still a need to further improve the devices using hot balloon technology for treatment of *i.a.* menorrhagia by improving its cost-efficiency and ensuring the safety for the patient. This is the object of the present invention.

### Summary of the invention

The object of the invention is achieved at least partly by an apparatus for performing heat treatment of a bodily cavity in a mammal, comprising a catheter intended for insertion into said bodily cavity, an inflatable balloon surrounding a distal end of said catheter in a liquid tight manner which balloon is adapted in use of said apparatus to be inflated and dilated in said bodily cavity, control means operatively connected to said balloon by at least one oscillation lumen and being provided with an oscillation assembly adapted to drive a heat transmitting fluid within said apparatus, and heating means adapted to heat said heat transmitting fluid, said control means being adapted to control pressure and temperature of said heat transmitting fluid, wherein said heating means have a heat emitting portion and a heat receiving portion which is use of the apparatus are positioned in thermal contact with each other, and which are separable from each other.

By the division of the heating means into a heat emitting portion and a heat receiving portion which are separable, two units may be produced having each its own internal circuitry for heat and possibly other supplies. By such a division of the apparatus the heat production may be performed in one unit, which consequently contains the devices for this production, and the produced heat may be transmitted to the other unit. The unit receiving the produced heat may therefore be simplified in design and shielded off from internal contact with the other unit. Hence, the unit comprising the heat receiving portion may be liquid tightly separated from the unit comprising the heat emitting portion and there is no risk for contamination of the liquid by the contact with the unit comprising the heat emitting portion. In the preferred embodiment of the invention the unit comprising the heat emitting portion is the control unit and the unit comprising the heat receiving portion is the catheter, but the scope of the claims is by no means limited to this embodiment. The unit comprising the heat receiving means, *i.e.* preferably the sterile catheter, can be produced at low cost and therefore becomes better adapted to be disposed after use if desired. Another advantage is that the amount of liquid circulating enclosed in the apparatus becomes small, which reduces serious burns if the balloon should burst. The heating up time will hence also be short and it has been tested that although the positioning of the heat emitting portion may be at a distance from the inflatable balloon the heat loss in the oscillation lumen is in principle negligible. Yet another advantage is that the unit comprising the heat receiving portion is not by itself connected to any power unit and therefore the patient is at no risk of being subjected to electric shock.

According to an aspect of the invention the apparatus is separable into a reusable unit and a disposable unit, said disposable unit being adapted to be sterile.

According to an aspect of the invention the heat emitting portion is positioned in said reusable unit and said heat receiving portion is positioned in said disposable unit. By the inventive apparatus it is possible to divide the heating means into a low cost disposable part, *i.e*. the heat receiving portion and preferably the catheter, and a high yield reusable part, *i.e.* the heat emitting portion and preferably the control unit. By locating the low cost means in the disposable catheter and the technically much more elaborated and expensive means for the heat production and heat emitting of the heating means in the reusable control unit the apparatus will be improved in terms of environmental friendliness and production costs.

According to an aspect of the invention the oscillation assembly comprises a fluid compartment in fluid connection with said oscillation lumen, a wall of said fluid compartment at least partly forming said heat receiving portion. This way a simply yet efficient structure is achieved.

According to an aspect of the invention the fluid compartment is connected to a proximal part of the oscillation lumen via a fluid inlet opening and is connected to a distal part of the oscillation lumen via a fluid outlet opening.

According to an aspect of the invention the fluid compartment has an at least partly conical shape, comprising a wider bottom portion and a narrower top portion as seen in a vertical direction. This way any air enclosed in the liquid and/or the compartment will ascend to the top portion where it collects and may be removed if required.

According to an aspect of the invention the fluid inlet opening is located near the bottom portion of the fluid compartment. When the liquid enters the compartment it hence enters close to the heat receiving portion and is thus immediately exposed to the heat.

According to an aspect of the invention at least a part of said bottom portion of the fluid compartment at least partly forms said heat receiving portion.

According to an aspect of the invention the heating means are heated by any of the methods chosen from the group of electric resistance heating, induction heating, microwave energy heating, and semi-conductive ceramic elements, such as PTC elements.

According to an aspect of the invention the heat emitting portion is a tempered surface and is in use of the apparatus positioned in mechanical contact with the heat receiving portion.

### Brief description of the drawings

The present invention is in the following described with the aid of the accompanying drawings, which are merely included as non-limiting representations of a preferred embodiment.
Fig. 1 shows an apparatus according to the invention,
fig. 2a shows in partial horizontal cross-section the control unit of fig. 1,
fig. 2b shows in cross-section the control unit of fig. 2a, and
fig. 3 shows in partial cross-section the catheter of fig. 1.

### Detailed description of a preferred embodiment of the invention

Throughout this application the words distal and proximal, respectively, are intended to refer to a situation as seen in relation to an operator or a user of the inventive apparatus.

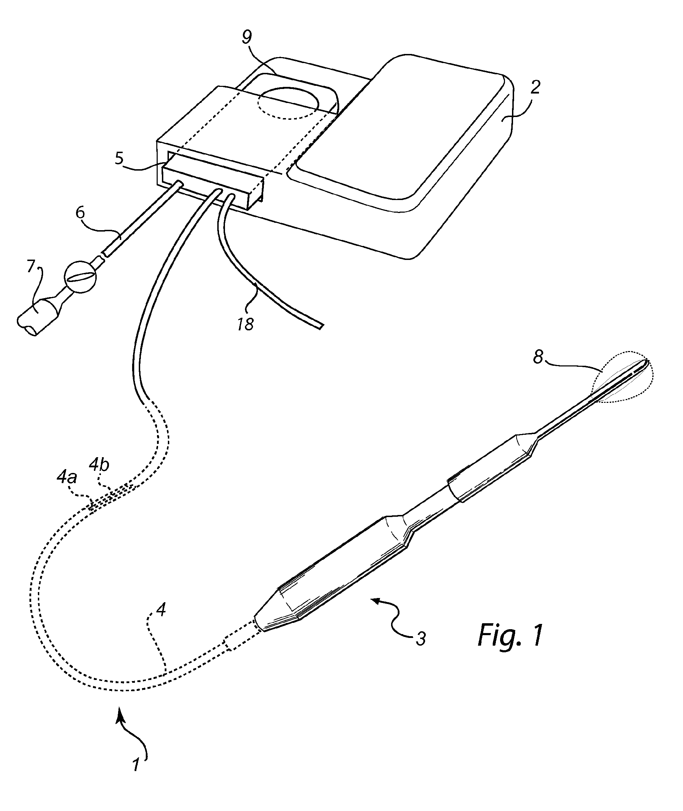

Reference is made to fig. 1 showing an apparatus for treatment of menorrhagia by heat balloon ablation which is one form of heat treatment. The apparatus comprises an ablation catheter 1 having a balloon catheter 3 with a balloon 8 and a conduit 4 containing a first lumen 4a and a second lumen 4b, a connector 5 in the proximal end of the conduit 4 and a control unit 2. The first lumen 4a is located within the second lumen 4b. The control unit 2 is supplying electrical power and any other supplies required to the apparatus in a known manner.

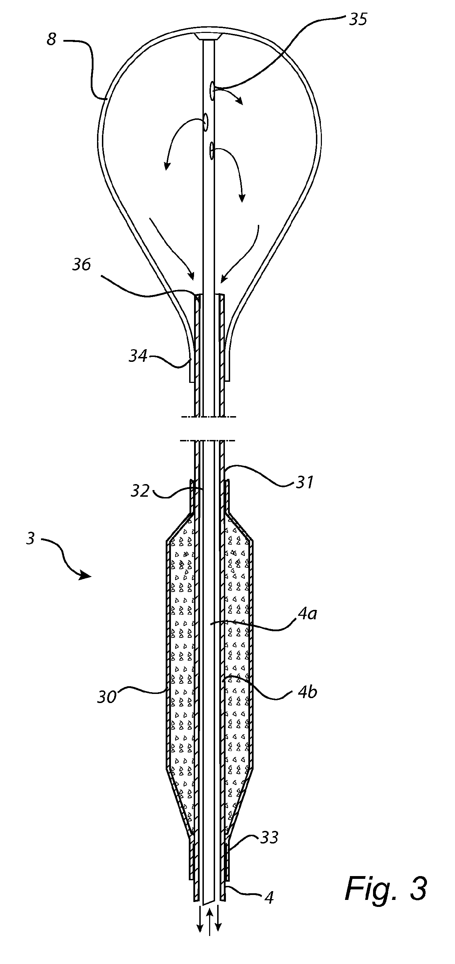

The first and second lumen 4a and 4b pass the connector 5 which is partly inserted in the control unit 2 and provides connection between the balloon catheter 3 and the control unit 2, and also via a conduit 6 from a separate syringe 7, for filling and regulating the pressure of a liquid within the apparatus. This liquid is one example of a heat transmitting fluid as claimed in the claims. The inner second lumen 4b is adapted to lead said liquid from the connector 5 to the balloon 8, and the outer first lumen 4b leads the liquid back from the balloon 8 towards the connector 5. Arrows indicating this are shown in fig. 3.

The control unit 2 comprises an oscillation assembly for creating a back-and-forth movement of a membrane in the connector 5, which in use induces circulation of the liquid from the membrane through a compartment 9 in the connector 5 and through the first lumen 4a. The liquid then passes through a second tube 32 in the balloon catheter 3 to the balloon 8, when the liquid returns through a first tube 31 connected to the second lumen 4b in and further back to the compartment 9 in the connector 5.

During use, the lumens 4a and 4b, the corresponding tubes 31 and 32 in the catheter 3, the balloon 8 and the compartment 9 in the connector 5 create a liquid tight circuit circulating the sterile liquid. A heater 29 in the control unit 2 provides heat at a controlled temperature to the liquid when circulating in the compartment 9.

The apparatus is divided into a reusable part which is intended for multi-use, and a disposable part. The reusable part comprises the control unit 2 and the disposable part comprises the ablation catheter 1, the connector 5 and the conduits 4 and 6.The disposable part is in the preferred embodiment intended to be sterile and used only once. The disposable part establishes a well defined and from the surrounding delimited internal system for supplies to the balloon 8 for the treatment of the patient. The disposable part meets the reusable part via the connector 5 which on its surface has contact portions suitable for yielding heat energy from the reusable part to the liquid contained in the disposable part. The claimed heat receiving portion is located on the connector's 5 surface, and the claimed heat emitting portion, i.e. the heater 29, is located within the pocket in the control unit 2 which is adapted to receive the connector 5. When the connector 5 is positioned within the pocket of the control unit 2 the heat receiving portion is located adjacent the heat emitting portion. The connector 5 is insertable into the control unit 2 and again removable therefrom.

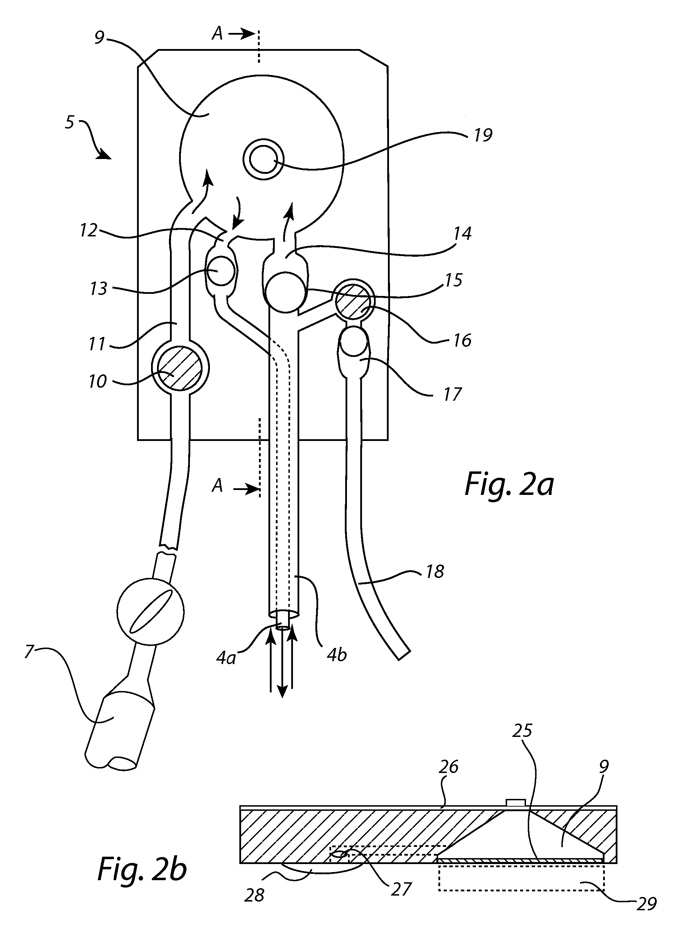

Fig. 2a shows in more detail the connector 5 with the conduit 6 for the inflation of the liquid from the syringe 7. A membrane 10 (positioned in the same plane as the figure and actuated by the control unit 2 in a known manner) induces circulation via a lumen 11 which is connected to the compartment 9. The compartment 9, which has a small volume, has one outlet 12 with a ball valve 13, and one inlet 14 with a ball valve 15. The outlet 12 is connected to the first lumen 4a and the inlet is connected to the second lumen 4b. The ball valves 13 and 15 are counteracting and in use transfer alternating back-and-forth pulsation of the liquid created of the membrane 10 to a stream of a liquid through the lumen 4a and further through the lumen 4b in the conduit 4 as earlier described. This is one embodiment of an oscillation assembly according to the claims. The arrows in the figure indicate a positive stroke with the ball valve 13 opened and the ball valve 15 closed. The figure also shows a membrane 16 (positioned in the same plane as the figure) for connection to a pressure sensor in the control unit 2 for indicating the pressure in the balloon 8. The membrane 16 is connected to a safety valve 17 with an exit 18. A valve for evacuation of air is depicted 19 and is positioned vertically above the compartment 9. The performance of the inventive apparatus is negatively affected if air is entering the system and it is therefore desired to be able to evacuate any air contained in the liquid. The bottom of the compartment 9 is heated from a heater located in the control unit 2 (shown in fig. 2b).

It is contemplated that the oscillation assembly and the conduit 4 may be designed to induce a pulsation to the liquid rather than a circulation. In such a case there is no need for a first and a second lumen within the conduit 4, but one single lumen for driving the liquid back-and-forth. Likewise, then one single tube is needed to connect the lumen to the balloon 8.

Fig. 2b shows a cross-sectional view A-A from fig. 2a of the connector 5. The compartment 9 has a circular and flat bottom portion 25 and an at least partially cone-shaped top portion and a lid 26 as seen in the vertical direction, creating a fluid tight space for containing fluid from which space the fluid exits to the lumen 4a during circulation. The bottom 25 is wider than the top. The bottom 25 of the fluid compartment 9 is heated by a heater depicted 29 (dashed line) inducing heat provided in the control unit 2. The heater 29 is a preferred embodiment of a heat emitting portion according to the claims, and said bottom 25 is a heat receiving portion correspondingly. A cut through the lumen 4a is depicted 27 and 28 depicts the membrane for back-and-forth movements. Preferably the part of the connector 5 containing the fluid compartment 9 is made at least partially of transparent material in order to allow visual control of the fluid flow during operation of the apparatus. It is hence possible to witness whether there is any air within the system. Air will be seen as a bubble at the vertical top of the compartment 9. The inlet 14 to the compartment 9 is located near the bottom 25 in order to maximise the heat transfer to the liquid. The compartment 9 may be designed differently, i.a. the compartment 9 may have a square bottom and a uniform height extension or a rounded design.

The incorporation of the heater 29 in the control unit 2, the heater 29 being in thermal contact with, but mechanically and electrically separated from the fluid compartment 9 in the connector 5, has many advantages which will be explained below. One out of several benefits of this embodiment in comparison with the devices such as in EP 1 139 944 B1 (Wallstén et al) earlier referred to is that the design of the balloon catheter can be considerably simplified in the absence of a heating system within it, and thus can be better adapted for mass production at much lower cost. This is also important from an environmental point of view if the sterile catheter is to be disposed after use. Furthermore, there is no way to contaminate the sterile liquid by the control unit 2. Also, the heater 29 in the non-disposable control unit 2 may be produced with improved quality and more efficient technology may be chosen since it is used again and again.

It should be understood that the wording "thermal contact" is intended to incorporate any manner of transmitting heat from the heat emitting portion in the control unit 2 to the heat receiving portion in the connector 5. Such manners may *i.a.* be electrical resistance heating, induction heating or microwave energy heating, but any manner that is found cost efficient and manageable in terms of size may be utilised.

Fig. 3 illustrates the simplified balloon catheter 3 partly in cross-section. The balloon catheter 3 comprises an elongate tubular body. In the proximal part is an insulated handle 30 provided for the operator of the ablation apparatus 1.

The two concentric tubes 31 and 32 in the centre of the catheter are in the proximal part 33 of the catheter attached to the conduit 4, thereby creating a continuation of the lumens 4a and 4b. At the distal end of the tube 32, which is longer than tube 31, is the inflation balloon 8 fixed. The collar of the balloon 34 is fluid tightly fixed to the distal part of the tube 31. In the distal part of the inner tube 32 are holes 35 provided creating inlets. Outlets 36 from the inner of the balloon are provided in the space between the concentric tubes 31 and 32. In use will the pressurised hot liquid circulate from the heating compartment 9 through lumen 4a in the conduit 4 and the tube 32, through the inlets 35, further through the balloon and the outlet 36 and back to the heating compartment. The heater 28 in the regulation unit has means for measuring and regulating the temperature of the fluid to a desired level which preferably is about 80 - 95 °C if a water-based liquid is used.

The embodiments of the invention that have been described above all have heating means positioned in the connector 5 and the control unit 2. However, the heating means may within of the scope of the claims be provided outside the control unit 2. Noteworthy however is that the two portions should be separable in order to achieve the effect simplification of the one unit and maintaining the major heat production parts in the other.

## Claims

1. Apparatus for performing heat treatment of a bodily cavity in a mammal, comprising a catheter intended for insertion into said bodily cavity, an inflatable balloon surrounding a distal end of said catheter in a liquid tight manner which balloon is adapted in use of said apparatus to be inflated and dilated in said bodily cavity, control means operatively connected to said balloon by at least one oscillation lumen and being provided with an oscillation assembly adapted to drive a heat transmitting fluid within said apparatus, and heating means adapted to heat said heat transmitting fluid, said control means being adapted to control pressure and temperature of said heat transmitting fluid, wherein said heating means have a heat emitting portion and a heat receiving portion which is use of the apparatus are positioned in thermal contact with each other, and which are separable from each other.

2. Apparatus according to claim 1, wherein said apparatus is separable into a reusable unit and a disposable unit, said disposable unit being adapted to be sterile.

3. Apparatus according to claim 2, wherein said heat emitting portion is positioned in said reusable unit and said heat receiving portion is positioned in said disposable unit.

4. Apparatus according to any one of the preceding claims, wherein said oscillation assembly comprises a fluid compartment in fluid connection with said oscillation lumen, a wall of said fluid compartment at least partly forming said heat receiving portion.

5. Apparatus according to claim 4, wherein said fluid compartment is connected to a proximal part of the oscillation lumen via a fluid inlet opening and is connected to a distal part of the oscillation lumen via a fluid outlet opening.

6. Apparatus according to claim 5, wherein said fluid compartment has an at least partly conical shape, comprising a wider bottom portion and a narrower top portion as seen in a vertical direction.

7. Apparatus according to claim 6, wherein said fluid inlet opening is located near the bottom portion of the fluid compartment.

8. Apparatus according to claim 6, wherein at least a part of said bottom portion of the fluid compartment at least partly forms said heat receiving portion.

9. Apparatus according to any one of the preceding claims, wherein said heating means are heated by any of the methods chosen from the group of electric resistance heating, induction heating, microwave energy heating, and semi-conductive ceramic elements.

10. Apparatus according to any one of the preceding claims, wherein said heat emitting portion is a tempered surface and is in use of the apparatus positioned in mechanical contact with the heat receiving portion.
